# FASCICULE DE BREVET EUROPEEN

(11) **EP 2 863 881 B1**
(45) Date de publication et mention de la délivrance du brevet: **25.05.2016**
(21) Numéro de dépôt: 13730005.9
(22) Date de dépôt: 29.05.2013
(51) Int. Cl.: A61K 8/81, A61K 47/32, A61Q 1/00, A61Q 5/00, A61Q 19/00, C08F 2/32, C08L 33/00

(54) **NOUVEAU LATEX INVERSE AUTO-INVERSIBLE EXEMPT DE TENSIOACTIF, SON UTILISATION COMME AGENT EPAISSISSANT DANS UNE COMPOSITION COSMETIQUE**
NEUARTIGER TENSIDFREIER SELBSTUMKEHRBARER UMKEHRLATEX UND VERWENDUNG DAVON ALS VERDICKUNGSMITTEL IN EINER KOSMETISCHEN ZUSAMMENSETZUNG
NOVEL SURFACTANT-FREE SELF-REVERSIBLE REVERSE LATEX, AND USE OF SAME AS A THICKENING AGENT IN A COSMETIC COMPOSITION

(30) Priorité: 25.06.2012 FR 1255994
(43) Date de publication de la demande: 29.04.2015
(73) Titulaire: SOCIETE D'EXPLOITATION DE PRODUITS POUR LES INDUSTRIES CHIMIQUES SEPPIC, 75007 Paris (FR)
(72) Inventeur: MALLO, Paul, 78110 Le Vésinet (FR); BRAUN, Olivier, 42170 St Just St Rambert (FR)
(74) Mandataire: Conan, Philippe Claude
(86) Numéro de dépôt international: PCT/FR2013/051205
(87) Numéro de publication internationale: WO 2014/001668

(56) Documents cités:
- DE-A1- 19 625 810
- FR-A1- 2 961 513

## Description

La présente demande concerne des latex inverse eau dans huile auto-inversibles, leur procédé de préparation et leur application en tant qu'épaississant et/ou émulsionnant de produits de soins de la peau, du cuir chevelu et des cheveux ou pour la fabrication de préparations cosmétiques, dermopharmaceutiques ou pharmaceutiques.

L'industrie cosmétique et l'industrie pharmaceutique utilisent très régulièrement des polymères épaississants synthétiques pour augmenter la viscosité des crèmes, des émulsions et de diverses solutions topiques. Les polymères épaississants synthétiques actuellement utilisés en ces domaines, se présentent sous deux formes physiques, la forme poudre et la forme liquide pour laquelle le polymère est dispersé dans une huile à l'aide d'agents tensioactifs et que l'on appelle couramment latex inverse.

Les polymères épaississants sous forme de poudre, les plus connus sont les polymères à base d'acide acrylique ou les copolymères à base d'acide acrylique et de ses esters. On citera par exemple, les polymères commercialisés sous les noms CARBOPOL™ et PEMULEN™. Ils sont décrits notamment dans les brevets américains US 5, 373,044, US 2, 798,053 et dans le brevet européen EP 0 301 532.

En cosmétique, on utilise aussi et toujours sous forme de poudre, des homopolymères ou copolymères à base d'acide 2-acrylamido-2-méthyl propanesulfonique. Ces polymères épaississants sont commercialisés sous le nom ARISTOFLEX™ et décrits notamment dans les brevets européens EP 0 816 403, EP 1 116 733 et EP 1 069 142. Il y a aussi le polymère commercialisé sous le nom SEPIMAX ZEN™ qui est un copolymère divulgué dans les demandes internationales publiées sous les numéros WO 2008/087326 et WO 2011/030044. Ces épaississants sous forme de poudre sont obtenus par polymérisation précipitante ; le (ou les) monomère(s) est (ou sont) mis en solution dans un solvant organique type benzène, acétate d'éthyle, cyclohexane, ou tertio-butanol.

Ces épaississants ont l'avantage de n'être constitués que du polymère lui-même. Le plus souvent ils ne contiennent aucune molécule tensioactive. Mais ils ont les inconvénients inhérents aux poudres : formation de poussière, temps de dissolution importants, manutention délicate.

L'industrie cosmétique utilise aussi très largement des épaississants sous forme de latex inverses et notamment ceux commercialisés par la demanderesse. On citera par exemple, les épaississants SEPIGEL™ 305, SIMULGEL™ 600, SIMULGEL ™ EG, SIMULGEL ™ NS, SIMULGEL ™ A, SEPIPLUS™ 400, ™ 265 et SEPIPLUS™ TM S. Ces épaississants sont obtenus par polymérisation en émulsion inverse. Ils présentent l'avantage d'être plus aisément manipulables et se dispersent très rapidement dans l'eau. De plus, ces produits développent des performances épaississantes remarquablement élevées ; ces performances sont probablement la conséquence du procédé pour leur préparation, une réaction de polymérisation en phase dispersée, qui conduit à des polymères de très hauts poids moléculaires. Néanmoins, ces épaississants sous forme de latex inverse contiennent une huile et surtout plusieurs agents tensioactifs qui peuvent parfois induire des réactions d'intolérance cutanée sur des sujets particulièrement sensibles. La présence de ces agents tensioactifs ne permet pas non plus l'utilisation des latex inverses pour épaissir des formules « surfactant free ».

En fait les latex inverses contiennent généralement au moins deux agents tensio-actifs :
- Au moins un agent tensio-actif de bas HLB qui permet de réaliser l'émulsion eau dans huile et d'en assurer la stabilité pendant la réaction de polymérisation. Les esters de sorbitan en sont de bons exemples.
- Au moins un agent tensio-actif de haut HLB qui permet de rendre le latex inverse final (résultat de la polymérisation en émulsion) auto-inversible. Cela signifie que lors de l'introduction de l'agent épaississant dans la phase aqueuse, on obtient aisément la dispersion et le gonflement des chaînes polymériques dans l'eau. Les polysorbates ou les alcools poly-éthoxylés en sont des bons exemples.

Des latex inverses épaississants sans tensio-actif de haut HLB sont divulgués dans la demande de brevet européen publiée sous le numéro EP 0 917 869. Néanmoins cette solution est très imparfaite car le latex épaississant reste stabilisé à l'aide d'au moins un tensioactif de bas HLB. De plus pour obtenir une bonne inversion du latex inverse il est nécessaire d'ajouter directement dans la formule cosmétique un agent tensioactif de haut HLB type polysorbate ou alcools poly-éthoxylés.

La demande brevet britannique publiée sous le numéro GB 1 499 731 divulgue la synthèse de copolymères stabilisants du type méthacrylate de stéaryle/acide méthacrylique et autre copolymères du même type. Des latex inverses comprenant ces copolymères sont décrits dans la demande de brevet européen publiée sous le numéro EP 0 161 038. La demande de brevet européen EP 0 049 766 divulgue l'utilisation de d'autres copolymères stabilisants. Mais on observe que les copolymères stabilisants sont souvent associés à des tensio-actifs classiques de bas HLB et surtout qu'un tensio-actif de haut HLB, de type nonylphénol éthoxylé est incorporé dans le latex inverse pour le rendre auto-inversible.

Les inventeurs ont cherché à développer de nouveaux latex inverses sans tensioactifs, qui soient stables et auto-inversibles.

Selon un premier aspect, l'invention a pour objet une composition sous forme d'un latex inverse auto-inversible comprenant pour 100% de sa masse :
**a) - De 25% massique à 80% massique** d'un polyélectrolyte anionique (P) réticulé, issu de la polymérisation pour 100% molaire :
   **i) -** d'une proportion molaire supérieure ou égale à 30% et inférieure ou égale à 95% d'unités monomériques issues d'au moins un monomère comportant une fonction acide fort, libre partiellement ou totalement salifiée ;
   **ii**) - **optionnellement** d'une proportion molaire supérieure ou égale à 10% et inférieure ou égale à 65% d'unités monomériques issues d'au moins un monomère neutre ;
   **iii**) - **optionnellement** d'une proportion molaire supérieure ou égale à 5% et inférieure ou égale à 65% d'unités monomériques issues d'au moins un monomère comportant une fonction acide faible, libre partiellement ou totalement salifiée ;
      et
   **iv) -** d'une proportion molaire supérieure à 0% et inférieure ou égale à 5% d'au moins un monomère de réticulation diéthylénique ou polyéthylénique ;
**b) - De 0,5% à 10% massique** d'un terpolymère de méthacrylate de stéaryle, de méthacrylate de béhènyle pentacosaéthoxylé et de N,N-diméthyl acrylamide, issu de la polymérisation pour 100% molaire de ;
   - 80% molaire à 95% molaire de méthacrylate de stéaryle,
   - 2,5% molaire à 10% molaire de N,N-diméthyl acrylamide et
   - 2,5% molaire à 10% molaire de méthacrylate de béhènyle pentacosaéthoxylé,
**c) - De 5% massique à 40% massique** d'au moins une huile, et
**d) - De 0,1% massique à 40% massique** d'eau.

L'invention a donc pour objet la composition telle que définie ci-dessus, se caractérisant notamment par le fait qu'elle ne comprend en son sein ni d'agents tensioactifs de type huile dans eau, ni d'agents tensioactifs de type eau dans huile.

Par méthacrylate de béhènyle pentacosaéthoxylé, on désigne le composé de formule (I) :

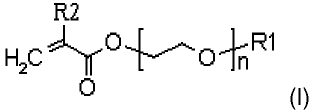

Dans laquelle dans laquelle R1 représente le radical docosanyle, R2 représente le radical méthyle et n est égal à 25.

Dans la composition objet de la présente invention, la phase huile est constituée soit par une huile minérale commerciale contenant des hydrocarbures saturés comme les paraffines, les isoparaffines ou les cycloparaffines présentant à température ambiante, une densité entre 0,7 et 0,9 et un point d'ébullition supérieur à environ 250°C, telle que par exemple l'ISOPAR™ M, le MARCOL™ 52 ou le MARCOL™ 82, commercialisés par EXXON CHEMICAL, soit par une huile végétale comme le squalane d'origine végétale, soit par une huile de synthèse tel que le polyisobutène hydrogéné ou le polydécène hydrogéné, soit par des esters d'acides gras avec le glycérol, qu'il s'agisse de mono-esters, de di-esters ou de triesters, soit par un mélange de plusieurs de ces huiles. Le MARCOL™ 52 est une huile commerciale répondant à la définition des huiles de vaseline du Codex français. C'est une huile blanche minérale conforme aux réglementations FDA 21 CFR 172.878 et CFR 178.3620 (a) et elle est inscrite à la Pharmacopée des USA, US XXIII (1995) et à la Pharmacopée européenne (1993).

Selon un autre aspect particulier, dans le polyélectrolyte P de la composition objet de la présente invention, la fonction acide fort des monomères en comportant est notamment la fonction acide sulfonique. Lesdits monomères sont par exemple l'acide styrènesulfonique libre, partiellement ou totalement salifié ou, plus particulièrement, l'acide 2-méthyl 2-[(1-oxo 2-propènyl) amino] 1-propanesulfonique (dénommé aussi acide 2-acrylamido 2-méthyl propanesulfonique) libre, partiellement ou totalement salifiée.

Selon un autre aspect particulier, dans le polyélectrolyte P compris dans la composition objet de la présente invention, la fonction acide faible des monomères en comportant est notamment la fonction acide carboxylique libre ou partiellement salifiée. Lesdits monomères sont par exemple l'acide acrylique, l'acide méthacrylique, l'acide itaconique, l'acide maléique, l'acide 3-méthyl 3-[(1-oxo 2-propènyl) amino] butanoïque ou l'acrylate de β-carboxy éthyle de formule :

CH₂=CH-C(=O)-O-CH₂-CH₂-C(=O)-OH,

lesdits acides carboxyliques étant libres ou partiellement salifiés. Il s'agit plus particulièrement de l'acide acrylique ou de l'acide méthacrylique libre ou partiellement salifié.

Pour les monomères à fonction acide fort ou à fonction acide faible, le terme salifié indique qu'il s'agit de sels de métaux alcalins tels que les sels de sodium ou de potassium, les sels de bases azotées comme, ou le sel d'ammonium.

Selon un autre aspect particulier, dans le polyélectrolyte P de la composition objet de la présente invention, le monomère neutre est choisi l'acrylamide, le N-méthyl acrylamide, le N,N-diméthyl acrylamide, le N,N-diéthyl acrylamide, le N-isopropyl acrylamide, l'acrylate de (2-hydroxy éthyle), l'acrylate de (2,3-dihydroxy propyle), le méthacrylate de (2-hydroxy éthyle) le méthacrylate, de (2,3-dihydroxy propyle), la N-vinyl pyrrolidone, le N-[2-hydroxy-1,1-bis(hydroxyméthyl) éthyl] propènamide, l'acrylate de (2-hydroxy éthyle) ou le N-(2-hydroxy éthyl) acrylamide)-.

Selon un autre aspect particulier, le terpolymère de méthacrylate de stéaryle, de méthacrylate de béhènyle pentacosaéthoxylé et de N,N-diméthyl acrylamide, présent dans la composition objet de la présente invention est choisi parmi les terpolymères suivants :
- Terpolymère de méthacrylate de stéaryle, de méthacrylate de béhènyle pentacosaéthoxylé et de N,N-diméthyl acrylamide [ratio molaire (A)/(B) /(C) = 87/5/8];
- Terpolymère de méthacrylate de stéaryle, de méthacrylate de béhènyle pentacosaéthoxylé et de N,N-diméthyl acrylamide [ratio molaire (A)/(B)/(C) = 80/5/15] ;
- Terpolymère de méthacrylate de stéaryle, de méthacrylate de béhènyle pentacosaéthoxylé et de N,N-diméthyl acrylamide [ratio molaire (A)/(B) /(C) = 85/10/5] ;
- Terpolymère de méthacrylate de stéaryle, de méthacrylate de béhènyle pentacosaéthoxylé et de N,N-diméthyl acrylamide [ratio molaire (A)/(B)/(C) = 80/10/10] ;
- Terpolymère de méthacrylate de stéaryle, de méthacrylate de béhènyle pentacosaéthoxylé et de N,N-diméthyl acrylamide [ratio molaire (A)/(B)/(C) = 90/5/5] ; ou du ;
- Terpolymère de méthacrylate de stéaryle, de méthacrylate de béhènyle pentacosaéthoxylé et de N,N-diméthyl acrylamide [ratio molaire (A)/(B)/(C) = 95/2,5/2,5)].

Tous ces terpolymères sont décrits dans la demande internationale publiée sous le numéro WO 2011/161349.

Selon un autre aspect particulier, dans la composition objet de la présente invention, le ratio massique entre le terpolymère de méthacrylate de stéaryle, de méthacrylate de béhènyle pentacosaéthoxylé et de N,N-diméthyl acrylamide et le polyélectrolyte P anionique est supérieur ou égal à 1/100 et inférieur ou égal à 1/5. Il est plus particulièrement supérieur ou égal à 5/100 et inférieur ou égal à 1/5.

L'invention a plus particulièrement pour objet composition sous forme d'un latex inverse auto-inversible comprenant pour 100% de sa masse :
**a) - De 25% massique à 50% massique** d'un polyélectrolyte anionique (P) branché ou réticulé, issu de la polymérisation pour 100% molaire :
   **i) -** d'une proportion molaire supérieure ou égale à 30% et inférieure ou égale à 95% d'unités monomériques issues d'au moins un monomère comportant une fonction acide fort, libre partiellement ou totalement salifiée ;
   **ii**) - d'une proportion molaire supérieure ou égale à 10% et inférieure ou égale à 65% d'unités monomériques issues d'au moins un monomère neutre ;
      et
   **iv) -** d'une proportion molaire supérieure à 0% et inférieure ou égale à 5% d'au moins un monomère de réticulation diéthylénique ou polyéthylénique ;
**b) - De 1% à 5% massique** d'un terpolymère de méthacrylate de stéaryle, de méthacrylate de béhènyle pentacosaéthoxylé et de N,N-diméthyl acrylamide, issu de la polymérisation pour 100% molaire;
   de 80% molaire à 95% molaire de méthacrylate de stéaryle,
   de 2,5% molaire à 10% molaire de N,N-diméthyl acrylamide, et
   de 2,5% molaire à 10% molaire de méthacrylate de béhènyle pentacosaéthoxylé,
**c) - De 10% massique à 30% massique** d'au moins une huile, et
**d) - De 20% massique à 40% massique** d'eau.

L'invention a plus particulièrement pour objet composition sous forme d'un latex inverse auto-inversible comprenant pour 100% de sa masse :
**a) - De 50% massique à 80% massique** d'un polyélectrolyte anionique (P) branché ou réticulé, issu de la polymérisation pour 100% molaire :
   **i) -** d'une proportion molaire supérieure ou égale à 30% et inférieure ou égale à 95% d'unités monomériques issues d'au moins un monomère comportant une fonction acide fort, libre partiellement ou totalement salifiée ;
   **ii**) - d'une proportion molaire supérieure ou égale à 10% et inférieure ou égale à 65% d'unités monomériques issues d'au moins un monomère neutre ;
      et
   **iv) -** d'une proportion molaire supérieure à 0% et inférieure ou égale à 5% d'au moins un monomère de réticulation diéthylénique ou polyéthylénique ;
**b) - De 1% à 10% massique** d'un terpolymère de méthacrylate de stéaryle, de méthacrylate de béhènyle pentacosaéthoxylé et de N,N-diméthyl acrylamide, issu de la polymérisation pour 100% molaire :
   de 80% molaire à 95% molaire de méthacrylate de stéaryle,
   de 2,5% molaire à 10% molaire de N,N-diméthyl acrylamide, et
   de 2,5% molaire à 10% molaire de méthacrylate de béhènyle pentacosaéthoxylé,
**c) - De 10% massique à 40% massique** d'au moins une huile, et
**d**) **- Au plus 5% massique** d'eau.

La composition selon l'invention peut également contenir divers additifs tels que des agents complexants, des agents de transfert ou des agents limiteurs de chaîne.

Selon un autre aspect de la présente invention, celle-ci a pour objet un procédé de préparation de la composition telle que définie précédemment, caractérisé en ce que :
a) l'on émulsionne une phase aqueuse (A) contenant les monomères et les éventuels additifs hydrophiles, dans une phase organique (O) contenant ledit terpolymère de méthacrylate de stéaryle, de méthacrylate de béhènyle pentacosaéthoxylé et de N,N-diméthyl acrylamide, l'huile ou le mélange d'huiles un mélange constitué des huiles destinées à être présente dans la composition finale et si nécessaire une ou plusieurs huiles volatiles ainsi que les éventuels additifs hydrophobes,
b) l'on amorce la réaction de polymérisation par introduction dans l'émulsion formée en a), d'un initiateur de radicaux libres puis on la laisse se dérouler, et si désiré
c) l'on concentre par distillation le milieu réactionnel issu de l'étape b), jusqu'à élimination complète de ladite huile volatile ;

Les huiles volatiles appropriées à la mise en oeuvre du procédé tel que défini ci-dessus, sont par exemple des isoparaffines légères comportant de 8 à 13 atomes de carbone comme par exemple celles vendues sous les noms ISOPAR™ G, ISOPAR™ L ou ISOPAR™ H ou ISOPAR™ J.

Selon une mise en oeuvre préférée du procédé tel que défini précédemment, la réaction de polymérisation est amorcée par un couple oxydoréducteur, tel que le couple hydroperoxyde de cumène - métabisulfite de sodium, à une température inférieure ou égale à 10°C, puis conduite soit de manière quasi-adiabatique jusqu'à une température supérieure ou égale à 40°C, plus particulièrement supérieure ou égale à 50°C, soit en contrôlant l'évolution de la température.

L'invention a aussi pour objet, l'utilisation du terpolymère de méthacrylate de stéaryle, de méthacrylate de béhènyle pentacosaéthoxylé et de N,N-diméthyl acrylamide, issu de la polymérisation pour 100% molaire :
de 80% molaire à 95% molaire de méthacrylate de stéaryle,
de 2,5% molaire à 10% molaire de N,N-diméthyl acrylamide et
de 2,5% molaire à 10% molaire de méthacrylate de béhènyle pentacosaéthoxylé, comme agent stabilisant de l'émulsion eau dans huile comprenant l'ensemble des constituants nécessaires à la préparation de la composition sous forme d'un latex inverse auto-inversible telle que définie précédemment, émulsion eau dans huile au sein de laquelle se déroule la réaction de polymérisation en émulsion inverse.

L'invention a aussi pour objet une composition topique cosmétique, dermopharmaceutique ou pharmaceutique, caractérisée en ce qu'elle comprend, comme agent épaississant et/ou émulsionnant, une quantité efficace de la composition telle que définie précédemment.

Une composition topique selon l'invention, destinée à être appliquée sur la peau, sur le cuir chevelu, sur les cheveux ou les muqueuses de l'homme ou de l'animal, peut consister en une émulsion topique comprenant au moins une phase aqueuse et au moins une phase huile, se présentant sous la forme d'une émulsion eau dans huile, ou huile dans eau, ou eau dans huile dans eau, ou huile dans eau dans huile. Plus particulièrement, cette émulsion topique peut consister en une émulsion fluide, telle un lait ou un gel fluide. La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau, du cuir chevelu et des muqueuses. Dans ce dernier cas, la composition topique comporte alors un principe actif qui peut par exemple consister en un agent anti-inflammatoire, un myorelaxant, un antifongique ou un antibactérien.

Lorsque la composition topique est utilisée en tant que composition cosmétique destinée à être appliquée sur la peau, sur le cuir chevelu, sur les cheveux ou les muqueuses, elle peut ou non comporter un principe actif, par exemple un agent hydratant, un agent bronzant, un filtre solaire, un antirides, un agent à visée amincissante, un agent antiradicalaire, un agent antipelliculaire, un agent anti-acnéique ou un antifongique.

Plus particulièrement, comme exemples de principes actifs éventuellement présents dans la composition topique selon l'invention, on peut citer : les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme par exemple l'ascorbyl glucoside), le tocophérol (vitamine E) et ses esters (comme par exemple l'acétate de tocophérol), les vitamines B3 ou B10 (niacinamide et ses dérivés) ; les composés montrant une action éclaircissante ou dépigmentante de la peau comme par exemple le SEPIWHITE™MSH, l'arbutine, l'acide koji-que, l'hydroquinone, le VEGEWHITE™, la GATULINE™, le SYNERLIGHT™, le BIOW-HITE™, le PHYTOLIGHT™, le DERMALIGHT™, la CLARISKIN™, le MELASLOW™, le DERMAWHITE™, l'ETHIOLINE, le MELAREST™, le GIGAWHITE™, l'ALBATINE™, le LUMISKIN™ ; les composés montrant une action apaisante comme le SEPICALM™ S, l'allantoïne et le bisabolol ; les agents anti-inflammatoires ; les composés montrant une action hydratante comme par exemple l'urée, les hydroxyurées, le glycérol, les polyglycérols, le glycérolglucoside, le diglycérolglucoside, les polyglycérylglucosides , l'érythrityl-glucoside, le xylityl glucoside, le sorbityl-glucoside, le sorbitol, la composition commercialisée sous l'appellation AQUAXYL™ ; les extraits végétaux riches en polyphénols comme par exemple les extraits de raisin, les extraits de pin, les extraits de vin, les extraits d'olives ; les composés montrant une action amincissante ou lipolytique comme la caféine ou ses dérivés, l'ADIPOSLIM™, l'ADIPOLESS™ ; les protéines N-acylées ; les peptides N-acylés comme par exemple le MATRIXIL™ ; les acides aminés N-acylés ; les hydrolysâts partiels de protéines N-acylés ; les acides aminés ; les peptides ; les hydrolysâts totaux de protéines ; les extraits de soja, par exemple la Raffermine™ ; les extraits de blé par exemple la TENSINE™ ou la GLIADINE™ ; les extraits végétaux, tels que les extraits végétaux riches en tanins, les extraits végétaux riches en isoflavones ou les extraits végétaux riches en terpènes ; les extraits d'algues d'eau douce ou marines ; les extraits marins en général comme les coraux ; les cires essentielles ; les extraits bactériens ; les céramides ; les phospholipides ; les composés montrant une action antimicrobienne ou une action purifiante, comme par exemple le LIPACIDE™ C8G, le LIPACIDE™ UG, le SEPICONTROL™ A5 ; l'OCTOPIROX™ ou le SENSIVA™ SC50 ; les composés montrant une propriété énergisante ou stimulante comme le Physiogényl™, le panthénol et ses dérivés comme le SEPICAP™ MP ; les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™ ;
les actifs anti-photo vieillissement ; les actifs protecteurs de l'intégrité de la jonction dermo-épidermique ; les actifs augmentant la synthèse des composants de la matrice extracellulaire comme par exemple le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme par exemple les dérivés de l'acide nicotinique) ou des produits créant une sensation de « fraîcheur » sur la peau (comme par exemple le menthol et des dérivés) ; les actifs améliorant la microcirculation cutanée, par exemple les veinotoniques ; les actifs drainants ; les actifs à visée décongestionnante comme par exemple les extraits de ginko biloba, de lierre, de marron d'inde, de bambou, de ruscus, de petit houx, de *centalla asiatica*, de fucus, de romarin, de saule ;les agents de bronzage ou de brunissement de la peau, comme la dihydroxyacétone, l'isatine, l'alloxane, la ninhydrine, le glycéraldéhyde, l'aldéhyde mésotartrique, la glutaraldéhyde ou l'érythrulose.

Par quantité efficace, on signifie que la composition topique selon l'invention comporte une quantité suffisante de latex inverse selon l'invention pour provoquer une modification de sa rhéologie. La composition topique selon l'invention comporte habituellement entre 0,1% et 10% en poids dudit latex inverse auto-inversible défini ci-dessus. Le pH de la composition topique est généralement compris entre 3 et 9.

La composition topique peut en outre comporter des composés classiquement compris dans ce type de compositions, par exemple des agents épaississants et/ou gélifiants, des agents stabilisants, des composés filmogènes, des solvants et co-solvants, des agents hydrotropes, des agents plastifiants, des agents opacificants, des agents nacrants, des agents surgraissants, des séquestrants, des agents chélatants, des agents antioxydants, des agents conditionneurs, des agents déodorants, des parfums, des huiles essentielles, des conservateurs, des colorants, des émollients, des particules procurant un effet visuel ou destinées à l'encapsulation d'actifs, des particules exfoliantes, des agents de texture, des azurants optiques, des repellents pour les insectes., des filtres solaires, des charges minérales ou des pigments.

Comme exemples d'agents épaississants et/ou gélifiants éventuellement présents dans la composition topique selon l'invention, on peut citer les hydrocolloïdes d'origine végétale ou biosynthétique, par exemple la gomme de xanthane, la gomme de karaya, les carraghénates, les alginates ; les galactomannanes comme par exemple la gomme de tara, la gomme de guar, la gomme de fenugrec, la gomme de caroube, la gomme de casse ; les silicates ; la cellulose et ses dérivés ; l'amidon et ses dérivés hydrophiles ; les polyuréthanes.

Comme exemples d'agents déodorants éventuellement présents dans la composition topique selon l'invention, on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3 propanediol ; l'acide salicylique ; le bicarbonate de sodium ; les cyclodextrines ; les zéolithes métalliques ; le Triclosan™ ; le bromohydrate d'aluinium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples de solvants et de co-solvants éventuellement présents dans la composition topique selon l'invention, on peut citer l'eau, les solvants organiques comme par exemple le glycérol, le diglycérol, les oligomères du glycérol, l'éthylène glycol, le propylène glycol, le butylène glycol, l'hexylène glycol, le diéthylène glycol, le xylitol, l'érythritol, le sorbitol, les alcools hydrosolubles tels que l'éthanol, l'isopropanol ou le butanol, les mélanges d'eau et desdits solvants organiques.

Comme exemples de filtres solaires éventuellement présents dans la composition topique selon l'invention, on peut citer tous ceux figurant dans la directive cosmétique 76/768/CEE modifiée annexe VII.

Parmi les filtres organiques solaires éventuellement présents dans la composition topique selon l'invention, on peut citer la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ; la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl-α-cyano-β-phényle, le cinnamate de diparaméthoxy mono-2-éthylhexanoyl de glycéryle ; la famille des dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-carboxylate, la 2-hydroxy-4-n-octyloxybenzophénone, la 4-hydroxy-3-carboxybenzophénone ; le 3-(4'-méthylbenzylidène)-d,l-camphre, le 3-(benzylidène)-d,l-camphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy)-1,3,5-triazine, le 4,4-((6-(((1,1-diméthyléthyl) amino)carbonyl)phenyl)amino)-1,3,5-triazine-2,4-diyl diimino) bis-(2-éthylhexyl) ester de l'acide benzoïque, le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-t-butylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate ; la famille des polysiloxanes comme le malonate de benzylidène siloxane.

Parmi les filtres inorganiques solaires, également appelés "écrans minéraux", éventuellement présents dans la composition topique selon l'invention, on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

Parmi les huiles essentielles éventuellement présentes dans la composition topique selon l'invention, on peut citer les huiles essentielles riches en carbures sesquiterniques et terpéniques, comme par exemple les huiles essentielles de térébenthine, de genévrier, de citron ; les huiles essentielles riches en alcools, comme par exemple les huiles essentielles de coriandre, de rose ; les huiles essentielles riches en alcools et en esters, comme par exemple les huiles essentielles de lavande, de menthe ; les huiles essentielles riches en aldéhydes, comme par exemple les huiles essentielles de cannnelle, de citronelle ou d'eucalyptus ; les huiles essentielles riches en cétones, comme par exemple les huiles essentielles de sauge ou de camphrier ; les huiles essentielles riches en phénols, comme par exemple les huiles essentielles de thym, de girofle ; les huiles essentielles riches en éthers, comme par exemple les huiles essentielles d'anis vert, de badiane, de fenouil ; les huiles essentielles riches en péroxydes, comme par exemple les huiles essentielles de chénopode et d'ail ; les huiles essentielles sulfurées comme par exemple les huiles essentielles de crucifères et de Liliacées.

Selon encore un autre aspect, l'invention concerne l'utilisation de la composition sous forme de latex inverse auto-inversible, pour épaissir une composition cosmétique, dermopharmaceutique ou pharmaceutique comprenant au moins une phase aqueuse.

La composition selon l'invention est un substitut intéressant à celles vendues sous les noms SEPIGEL™ 305, SEPIGEL™ 501, SIMULGEL™ EG, SIMULGEL™ NS ou SIMULGEL™ 600 par la demanderesse, car elle présente aussi une bonne compatibilité avec les autres excipients utilisés pour la préparation de formulations telles que les laits, les lotions, les crèmes, les savons, les bains, les baumes, les shampooings ou les après-shampooings. Elle peut aussi être mise en oeuvre avec lesdits SEPIGEL ou SIMULGEL. Elle est notamment compatible avec les concentrés décrits et revendiqués dans les publications internationales WO 92/06778, WO 95/04592, WO 95/13863, WO 96/37285, WO 98/22207, WO 98/47610 ou dans FR 2734 496, avec les agents tensioactifs décrits dans WO 93/08204. Elle est particulièrement compatible avec le MONTANOV™ 68, le MONTANOV™ 82, le MONTANOV™ 202, le MONTANOV™ L, le MONTANOV™ 14 ou le MONTANOV™S. Elle peut également être utilisée dans des émulsions du type de celles décrites et revendiquées dans EP 0 629 396 et dans les dispersions aqueuses cosmétiquement ou physiologiquement acceptable avec un composé organopolysiloxane choisi, par exemple parmi ceux décrits dans WO 93/05762 ou dans WO 93/21316. Elle peut également être utilisée pour former des gels aqueux à pH acide cosmétiquement ou physiologiquement acceptable, tels que ceux décrit dans WO 93/07856 ; elle peut également être utilisée en association avec des celluloses non-ioniques, pour former par exemple des gels de coiffage tels que ceux décrits dans EP 0 684 024, ou encore en association avec des esters d'acides gras et de sucre, pour former des compositions pour le traitement du cheveu ou de la peau telles que celles décrites dans EP 0 603 019 ou encore dans les shampooings ou après-shampooings tels que décrits et revendiqués dans WO 92/21316 ou enfin en association avec un homopolymère anionique tels que le CARBOPOL™ pour former des produits de traitement des cheveux comme ceux décrits dans DE 195 23596 ou en association avec d'autres polymères épaississants.

La composition selon l'invention est également compatible avec les principes actifs tels que par exemple, les agents auto-bronzants comme le dihydroxyacétone (DHA) ou les agents anti-acné ; elle peut donc être introduite dans des compositions auto-bronzantes comme celles revendiquées dans EP0 715 845, EP 0 604 249, EP 0 576 188 ou dans 93/07902.

Elle est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561 ou dans WO 98/09611, et ce qui permet également son utilisation dans des compositions éclaircissantes de la peau humaine telles que celles décrites ou revendiquée dans WO2003/061768.

Lorsque la composition telle que définie précédemment est destinée au traitement de la peau et/ou du cuir chevelu et/ou des muqueuses, elle comprend plus particulièrement un latex inverse de polyélectrolyte anionique objet de la présente invention. Les latex inverse objet de la présente invention peuvent être utilisés comme épaississant de pâtes d'impression textile. Les exemples suivants illustrent la présente invention sans toutefois la limiter.

### Exemple A : Latex inverse auto-inversible d'un copolymère ATBSNa/HEA réticulé au MBA.

### Préparation phase aqueuse

On charge dans un bécher, sous agitation :
- 672,5g d'une solution commerciale à 55% massique du sel de sodium de l'acide 2-méthyle-2-[(1-oxo-2-propényl) amino] 1-propane sulfonique (ATBSNa),
- 20,8g d'acrylate de 2-hydroxy éthyle (HEA),
- 0,152g de méthylène bis(acrylamide) (MBA),
- 0,45g d'une solution commerciale à 40 % massique de diéthylènetriamine penta-acétate de sodium.

On ajuste le pH à 4 à l'aide de l'acide 2-méthyle-2-[(1-oxo-2-propényl) amino] 1-propanesulfonique puis on complète la phase aqueuse jusqu'à 700g avec de l'eau.

### Préparation de la phase organique

On mélange sous simple agitation dans un bécher :
- 220 g d'ISOPAR™ M (isoparaffine en C13/C14),
- 20 g d'une solution de méthacrylate de stéaryle/N,N-diméthyl acrylamide/ méthacrylate de béhényle éthoxylé à 25 moles en solution à 46 % dans l'Isopar M,
- 0,15 g de d'AIBN (azobis (isobutyronitrile).

On verse ensuite la phase aqueuse dans la phase huile sous agitation et on émulsifie en maintenant le bécher dans un bain de glace à l'aide d'une turbine type Ultra-turrax jusqu'à obtenir une viscosité d'environ 4600mPas (Brookfield RVT M4, V20). On place ensuite l'émulsion dans le réacteur de polymérisation. On effectue un barbotage d'azote pendant environ 45 minutes tout en refroidissement le réacteur jusqu'à environ 8°C. On ajoute alors 10 cm³ d'une solution à 0,17% (masse/volume) d'hydroperoxyde de cumène dans de l'ISOPAR™M, puis une solution aqueuse de métabisulfite de sodium à 0,27% (masse/masse) dans l'eau à raison de 0,5cm³/minute. L'introduction est arrêtée en fin de d'exothermie. On obtient en fin de polymérisation, un latex inverse auto-inversible stable, dénommé par la suite : Latex inverse auto-inversible (1).

### Evaluation des propriétés

Viscosité du latex inverse auto-inversible (Brookfield RVT, M3, 20 rpm) : η = 1.800mPas
Viscosité du latex inverse auto-inversible mélangé à 2% massique dans l'eau (Brookfield RVT, axe 5 ; 5 rpm) : n = 158 000 mPa s .
Cette viscosité est obtenue sans ajout d'un tensioactif. De plus la vitesse d'inversion est de l'ordre de 2 minutes donc similaire à celle obtenue pour les latex inverses classiques.

### Exemple B : Latex inverse auto-inversible concentré d'un copolymère ATBSNa/HEA réticulé au MBA.

### Préparation phase aqueuse

On prépare la même phase aqueuse que celle de l'exemple A, à l'exception de la quantité en méthylène bis(acrylamide) qui est réduite à 0,028g.

### Préparation de la phase organique

On mélange sous simple agitation dans un bécher :
- 100g d'ISOPAR™ H
- 200g de triglycéride C8-C10
- 25g d'une solution à 46 % dans l'ISOPAR™ M de méthacrylate de stéaryle/N,N-diméthyl acrylamide/méthacrylate de béhényle éthoxylé à 25 moles en solution
- 0,15 g de d'AIBN (azobis (isobutyronitrile).

On verse la phase aqueuse dans la phase huile sous agitation et on émulsifie en maintenant le bécher dans un bain de glace à l'aide d'une turbine type Ultra-turrax jusqu'à l'obtention d'une valeur de viscosité d'environ (3000mPas) (Brookfield RVT M4, V20). On place ensuite l'émulsion dans le réacteur de polymérisation. On effectue un barbotage d'azote pendant environ 45 minutes tout en refroidissement le réacteur jusqu'à environ 8°C. On ajoute alors 10 cm³ d'une solution à 0,17% (masse/volume) d'hydroperoxyde de cumène dans du triglycéride C8-C10, puis une solution aqueuse de métabisulfite de sodium à 0,27% (masse/masse) dans l'eau à raison de 0,5cm³/minute. L'introduction est arrêtée en fin de d'exothermie. Puis une heure après la fin de la polymérisation, on met le réacteur sous vide partiel à hauteur d'environ 17 10³ Pa et on règle la température à 80° C de manière à retirer l'ISOPAR™ H et l'eau par évaporation. Après refroidissement on obtient un latex inverse auto-inversible concentré contenant environ 65% massique polyélectrolyte anionique, dénommé par la suite : Latex inverse auto-inversible (2). Ce latex est stable vis-à-vis des phénomènes de sédimentation et s'inverse spontanément dans l'eau.

### Evaluation des propriétés

Viscosité du latex inverse auto-inversible (Brookfield RVT, M3, 20 rpm) : η = 3.800mPas. Viscosité du latex inverse auto-inversible à 2% dans l'eau (Brookfield RVT, M5 ; 5 rpm) : η = 63 600 mPa s .

Cette viscosité est obtenue sans l'ajout de tensioactif. De plus la vitesse d'inversion est de l'ordre de 4 minutes donc similaire à celle obtenue pour les latex inverses concentrés.

Viscosité du latex inverse auto-inversible à 2% dans l'eau + 0,1% chlorure de sodium (Brookfield RVT, axe 5 ; 5 rpm) :
η = 31 000 mPa s.

### Exemples de formulations cosmétiques (proportions exprimées en pourcentages massiques)

**Exemple 1 Crème de soin**

| | |
|---|---|
| Cyclométhicone : | 10% |
| Latex inverse auto-inversible (2) : | 0,8% |
| MONTANOV ™ 68 : | 4,5% |
| Conservateur : | 0,65% |
| Lysine : | 0,025% |
| EDTA (sel disodique) : | 0,05% |
| Gomme de xanthane : | 0,2% |
| Glycérine : | 3% |
| Eau : | qsp 100% |

**Exemple 2 : Crème de soin**

| | |
|---|---|
| Cyclométhicone : | 10% |
| Latex inverse auto-inversible (1) : | 0,8% |
| MONTANOV™ 68 : | 4,5% |
| Perfluoropolyméthylisopropyléther : | 0,5% |
| Conservateur : | 0,65% |
| Lysine : | 0,025% |
| EDTA (sel disodique) : PEMULEN™ TR : | 0,05% |
| Glycérine : | 3% |
| Eau : | qsq 100% |

### Exemple 3 : Baume après-rasage

**FORMULE**

| | | |
|---|---|---|
| A | Latex inverse auto-inversible (1) : | 1,5% |
| | Eau : | qsq. 100% |
| B | MICROPEARL™ M100 : | 5,0% |
| | SEPICIDE™CI : | 0,50% |
| | Parfum : | 0,20% |
| | Ethanol à 95° : | 10,0% |

### MODE OPERATOIRE

Ajouter B dans A.

### Exemple 4 : Emulsion satinée pour le corps

**FORMULE**

| | | |
|---|---|---|
| A | SIMULSOL™ 165 : | 5,0% |
| | LANOL™ 1688 : | 8,50% |
| | Beurre de Karité : | 2% |
| | Huile de paraffine : | 6,5% |
| | LANOL™ 14 M : | 3% |
| | LANOL™ S : | 0,6% |
| B | Eau : | 66,2% |
| C | MICROPEARL™ M 100 : | 5% |
| D | Latex inverse auto-inversible (1) : | 3% |
| E | SEPICIDE™ CI | 0,3% |
| | SEPICIDE™ HB _{:} | 0,5% |
| | MONTEINE™ CA : | 1% |
| | Parfum : | 0,20% |
| | Acétate de vitamine E : | 0,20% |
| | pyrolidinonecarboxylate de sodium (agent hydratant) : | 1% |

### MODE OPERATOIRE

Ajouter C dans B, émulsionner B dans A à 70°C, puis ajouter D à 60°C puis E à 30°C.

### Exemple 5 : Lait corporel

**FORMULE**

| | | |
|---|---|---|
| A | SIMULSOL™ 165 : | 5,0% |
| | LANOL™ 1688 : | 12,0% |
| | LANOL™ 14 M : | 2,0% |
| | Alcool cétylique : | 0,3% |
| | SCHERCEMOL™ OP _{:} | 3% |
| B | Eau: | q.s.p. 100% |
| C | Latex inverse auto-inversible (2) : | 0,35% |
| D | SEPICIDE™ CI : | 0,2% |
| | SEPICIDE™HB : | 0,5% |
| | Parfum : | 0,20% |

### MODE OPERATOIRE

Emulsionner B dans A à 70°C, ajouter C, puis ajouter D à 60°C puis E à 30°C.

### Exemple 6 : Crème H/E

**FORMULE**

| | | |
|---|---|---|
| A | SIMULSOL™ 165 : | 5,0% |
| | LANOL™ 1688 : | 20,0% |
| | LANOL™ P : | 1,0% |
| B | Eau : | q.s.p. 100% |
| C | Latex inverse auto-inversible (2) : | 2,50% |
| D | SEPICIDE™ CI : | 0,20% |
| | SEPICIDE™ HB : | 0,30% |

### MODE OPERATOIRE

Introduire B dans A vers 75°C ; ajouter C vers 60°C, puis D vers 45°C.

### Exemple 7 : Gel solaire non gras

**FORMULE**

| | | |
|---|---|---|
| A | Latex inverse auto-inversible (1) : | 3.00% |
| | Eau: | 30% |
| B | SEPICIDE™ C : | 0,20% |
| | SEPICIDE™ HB : | 0,30% |
| | Parfum : | 0,10% |
| C | Colorant : | qs |
| | Eau: | 30% |
| D | MICROPEARL™ M 100 : | 3,00% |
| | Eau : | q.s.p. 100% |
| E | Huile de silicone : | 2,0% |
| | PARSOL™ MCX : | 5,00% |

### MODE OPERATOIRE

Introduire B dans A ; ajouter C puis D, puis E.

### Exemple 8 : Lait solaire

**FORMULE**

| | | |
|---|---|---|
| A | SEPIPERL™ N : | 3,0% |
| | Huile de sésame : | 5,0% |
| | PARSOL™ MCX : | 5,0% |
| | Carraghénane : | 0,10% |
| B | Eau : | q.s.p.100% |
| C | Latex inverse auto-inversible (1) : | 0,80% |
| D | Parfum : | q.s. |
| | Conservateur : | q.s. |

### MODE OPERATOIRE

Emulsionner B dans A à 75°C puis ajouter C vers 60°C, puis D vers 30°C et ajuster le pH si nécessaire.

### Exemple 9 : Gel de massage

**FORMULE**

| | | |
|---|---|---|
| A | Latex inverse auto-inversible (1) : | 3,5% |
| | Eau : | 20,0% |
| B | Colorant : | 2 gouttes/100g |
| | Eau: | q.s. |
| C | Alcool : | 10% |
| | Menthol : | 0,10% |
| D | Huile de silicone : | 5,0% |

### MODE OPERATOIRE

Ajouter B dans A ; puis ajouter au mélange, C puis D.

### Exemple 10 : Gel soin de massage

**FORMULE**

| | | |
|---|---|---|
| A | Latex inverse auto-inversible (2) : | 3,00% |
| | Eau: | 30% |
| B | SEPICIDE™ CI : | 0,20% |
| | SEPICIDE™ HB : | 0,30% |
| | Parfum : | 0,05% |
| C | colorant : | q.s. |
| | Eau : | q.s.p. 100% |
| D | MICROPEARL™ SQL : | 5,0% |
| | LANOL™ 1688 : | 2% |

### MODE OPERATOIRE

Préparer A ; additionner B, puis C, puis D.

### Exemple 11 : Gel coup d'éclat

**FORMULE**

| | | |
|---|---|---|
| A | Latex inverse auto-inversible (1) : | 4% |
| | Eau: | 30% |
| B | ELASTINE HPM : | 5,0% |
| C | MICROPEARL™ M 100 : | 3% |
| | Eau: | 5% |
| | SEPICIDE™ CI : | 0,2% |
| | SEPICIDE™ HB : | 0,3% |
| | Parfum : | 0,06% |
| | Pyrolidinonecarboxylate de sodium à 50% : | 1% |
| | Eau : | q.s.p. 100% |

### MODE OPERATOIRE

Préparer A ; additionner B, puis C, puis D.

### Exemple 12 : Lait corporel

**FORMULE**

| | | | |
|---|---|---|---|
| A | SEPIPERL™ N : | | 3,0% |
| | | Triheptonate de glycérol : | 10,0% |
| B | | Eau : | q.s.p. 100% |
| C | | Latex inverse auto-inversible (1) : | 1,0% |
| D | Parfum : | | q.s. |
| | | Conservateur : | q.s. |

### MODE OPERATOIRE

Fondre A à environ 75°C. Emulsionner B dans A à 75°C puis ajouter C vers 60°C, puis D.

### Exemple 13 : Emulsion démaquillante à l'huile d'amandes douces

**FORMULE**

| | |
|---|---|
| MONTANOV™ 68 : | 5% |
| Huile d'amandes douces : | 5% |
| Eau : | q.s.p. 100% |
| Latex inverse auto-inversible (2) : | 0,3% |
| Glycérine : | 5% |
| Conservateur : | 0,2% |
| Parfum : | 0,3% |

### Exemple 14 : Crème hydratante pour peaux grasses

**FORMULE**

| | |
|---|---|
| MONTANOV™ 68 : | 5% |
| Octanoate de cétylstéaryle : | 8% |
| Palmitate d'octyle : | 2% |
| Eau : | q.s.p. 100% |
| Latex inverse auto-inversible (1) : | 0,6% |
| MICROPEARL™ M 100 : | 3,0% |
| Mucopolysaccharides : | 5% |
| SEPICIDE™ HB : | 0,8% |
| Parfum : | 0,3% |

### Exemple 15 : Baume après-rasage apaisant sans alcool

**FORMULE**

| | |
|---|---|
| Mélange de N-lauryl aminoacides : | 0,1% à 5% |
| Aspartate de magnésium et de potassium : | 0,002% à 0,5% |
| LANOL™ 99 : | 2% |
| Huile d'amandes douces : | 0,5% |
| Eau : | q.s.p. 100% |
| Latex inverse auto-inversible (2) : | 3% |
| SEPICIDE™ HB : | 0,3% |
| SEPICIDE™ CI : | 0,2% |
| Parfum : | 0,4% |

### Exemple 16 : Crème aux AHA pour peaux sensibles

**FORMULE**

| | |
|---|---|
| Mélange de N-lauryl aminoacides : | 0,1% à 5% |
| Aspartate de magnésium et de potassium : | 0,002% à 0,5% |
| LANOL™ 99 : | 2% |
| MONTANOV™ 68 : | 5,0% |
| Eau : | q.s.p. 100% |
| Latex inverse auto-inversible (1) : | 1,50% |
| Acide gluconique : | 1,50% |
| Triéthanolamine : | 0,9% |
| SEPICIDE™ NB : | 0,3% |
| SEPICIDE™ CI : | 0,2% |
| Parfum : | 0,4% |

### Exemple 17 : Soin apaisant après-soleil

**FORMULE**

| | |
|---|---|
| Mélange de N-lauryl aminoacides : | 0,1% à 5% |
| Aspartate de magnésium et de potassium : | 0,002% à 0,5% |
| LANOL™ 99 : | 10,0% |
| Eau : | q.s.p. 100% |
| Latex inverse auto-inversible (1) : | 2,50% |
| SEPICIDE™ HB : | 0,3% |
| SEPICIDE™ CI : | 0,2% |
| Parfum : | 0,4% |
| Colorant : | 0,03% |

### Exemple 18 : Lait démaquillant

**FORMULE**

| | |
|---|---|
| SEPIPERL™ N : | 3% |
| PRIMOL™ 352 : | 8,0% |
| Huile d'amandes douces : | 2% |
| Eau : | q.s.p. 100% |
| Latex inverse auto-inversible (1) : | 0,8% |
| Conservateur : | 0,2% |

### Exemple 19 : Lait corporel

**FORMULE**

| | |
|---|---|
| SEPIPERL™ N : | 3,5% |
| LANOL™ 37T : | 8,0% |
| SOLAGUM™ L : | 0,05% |
| Eau : | q.s.p. 100% |
| Benzophénone: | 2,0% |
| Diméthicone 350 cPs : | 0,05% |
| Latex inverse auto-inversible (1) : | 0,8% |
| Conservateur : | 0,2% |
| Parfum : | 0,4% |

**Exemple 20 : émulsion fluide à pH alcalin**

| | |
|---|---|
| MARCOL™ 82 : | 5,0% |
| NaOH | 10,0% |
| Eau : | q.s.p. 100% |
| Latex inverse auto-inversible (2) : | 1,5% |
| LANOL™ 84D : | 8,0% |
| LANOL™ 99 : | 5,0% |
| Eau : | q.s.p. 100% |

### Exemple 21 : Lait solaire

**FORMULE**

| | |
|---|---|
| SEPIPERL™ N : | 3,5% |
| LANOL™ 37T : | 10,0% |
| PARSOL™ NOX : | 5,0% |
| EUSOLEX™ 4360 : | 2,0% |
| Eau : | q.s.p. 100% |
| Latex inverse auto-inversible (1) : | 1,8% |
| Conservateur : | 0,2% |
| Parfum : | 0,4% |

### Exemple 22 : Gel contour des yeux

**FORMULE**

| | |
|---|---|
| Latex inverse auto-inversible (1): | 2,0% |
| Parfum : | 0,06% |
| pyrrolidinonecarboxylate de sodium : | 0,2% |
| DOW CORNING™ 245 FLuid : | 2,0% |
| Eau : | q.s.p. 100% |

### Exemple 23 : Composition de soin non rincée

**FORMULE**

| | |
|---|---|
| Latex inverse auto-inversible (2) : | 1,5% |
| Parfum : | q.s. |
| Conservateur : | q.s. |
| DOW CORNING™ X2 8360 : | 5,0% |
| DOW CORNING™ Q2 1401 : | 15,0% |
| Eau : | q.s.p. 100% |

### Exemple 24 : Gel amincissant

| | |
|---|---|
| Latex inverse auto-inversible (1) : | 5% |
| Ethanol : | 30% |
| Menthol : | 0,1% |
| Caféine : | 2,5% |
| Extrait de ruscus : | 2% |
| Extrait de lierre : | 2% |
| SEPICIDE™ HB : | 1% |
| Eau : | q.s.p. 100% |

### Exemple 25 : Baume après-rasage apaisant sans alcool

**FORMULE**

| | | |
|---|---|---|
| A | LIPACIDE™ PVB : | 1,0% |
| | LANOL™ 99 : | 2,0% |
| | Huile d'amandes douces : | 0,5% |
| B | Latex inverse auto-inversible (1) : | 3,5% |
| C | Eau : | q.s.p. 100% |
| D | Parfum : | 0,4% |
| | SEPICIDE™ HB : | 0,4% |
| | SEPICIDE™ CI : | 0,2% |

### Exemple 26 : Gel rafraîchissant après-rasage

**FORMULE**

| | | |
|---|---|---|
| | LIPACIDE™ PVB : | 0,5% |
| | LANOL™ 99 : | 5,0% |
| | Latex inverse auto-inversible (2) : | 2,5% |
| B | eau : | q.s.p. 100% |
| C | MICROPEARL™ LM : | 0,5% |
| Parfum : | | 0,2% |
| SEPICIDE™ HB : | | 0,3% |
| SEPICIDE™ CI : | | 0,2% |

### Exemple 27 : Soin pour les peaux grasses

**FORMULE**

| | | |
|---|---|---|
| A | MICROPEARL™ M310 : | 1,0% |
| | Latex inverse auto-inversible (1) : | 5.0% |
| | Isononanoate d'octyle : | 4,0% |
| B | Eau : q.s.p. | 100% |
| C | SEPICONTROL™ A5 _{:} | 4,0% |
| | Parfum : | 0,1% |
| | SEPICIDE™ HB : | 0,3% |
| | SEPICIDE™ CI : | 0,2% |
| D | CAPIGEL™ 98 : | 0,5% |
| Eau: | | 10% |

### Exemple 28 : Crème aux AHA

**FORMULE**

| | | |
|---|---|---|
| A | MONTANOV™ 68 : | 5,0% |
| | LIPACIDE™ PVB : | 1,05% |
| | LANOL™ 99 : | 10,0% |
| B | Eau : | q.s.p. 100% |
| | Acide gluconique : | 1,5% |
| | Triéthanolamine : | 0,9% |
| C | Latex inverse auto-inversible (1) : | 1,5% |
| D Parfum: | | 0,4% |
| SEPICIDE™ HB : | | 0,2% |
| SEPICIDE™ CI : | | 0,4% |

### Exemple 29 : Autobronzant non gras pour visage et corps

**FORMULE**

| | | |
|---|---|---|
| A | LANOL™ 2681 : | 3,0% |
| | Latex inverse auto-inversible (1) | 2,5% |
| B | Eau : | q.s.p. 100% |
| | Dihydroxyacétone : | 3,0% |
| C | Parfum : | 0,2% |
| | SEPICIDE™ HB : | 0,8% |
| | Hydroxyde de sodium : | qs.pH = 5 |

### Exemple 30 : Lait solaire au monoï de Tahiti

**FORMULE**

| | | |
|---|---|---|
| A | Monoï de Tahiti : | 10% |
| | LIPACIDE™ PVB : | 0,5% |
| | Latex inverse auto-inversible (2) : | 2,2% |
| B | Eau : | q.s.p. 100% |
| C | Parfum : | 0,1% |
| | SEPICIDE™ HB : | 0,3% |
| SEPICIDE™ CI : | | 0,1% |
| Méthoxycinnamate d'octyle : | | 4,0% |

### Exemple 31 : Soin solaire pour le visage

**FORMULE**

| | | |
|---|---|---|
| A | Cyclométhicone et diméthiconol : | 4,0% |
| | Latex inverse auto-inversible (1) : | 3,5% |
| B | Eau : | q.s.p. 100% |
| C | Parfum : | 0,1% |
| SEPICIDE™ HB : | | 0,3% |
| SEPICIDE™ CI : | | 0,21% |
| Méthoxycinnamate d'octyle : | | 5,0% |
| Micatitane : | | 2,0% |
| Acide lactique : | | q.s.p.pH 6,5 |

### Exemple 32 Emulsion bronzante sans soleil

**FORMULE**

| | | |
|---|---|---|
| A | LANOL™ 99 : | 15% |
| | MONTANOV™ 68 : | 5,0% |
| | Paraméthoxycinnamate d'octyle : | 3,0% |
| B | Eau : | q.s.p. 100% |
| | Dihydroxyacétone : | 5,0% |
| | Phosphate monosodique : | 0,2% |
| C | Latex inverse auto-inversible (2) : | 0,5% |
| D | Parfum : | 0,3% |
| | SEPICIDE™ HB : | 0,8% |
| | Hydroxyde de sodium : | q.s. pH 7,5. |

### Exemple 33 : Gel brillance

| | |
|---|---|
| Latex inverse auto-inversible (1) : | 1,5% |
| Silicone volatile : | 25% |
| Monopropylèneglycol : | 25% |
| Eau déminéralisée : | 10% |
| Glycérine : | qsp.100% |

### Exemple 34 : Gel amincissant

| | |
|---|---|
| Latex inverse auto-inversible (2) : | 1,5% |
| Isononanoate d'isononyle : | 2% |
| Caféine : | 5% |
| Ethanol : | 40% |
| MICROPEARL™ LM : | 2% |
| Eau déminéralisée : | qsp. 100% |
| Conservateur parfum : | qs |

### Exemple 35 : Lait démaquillant

| | |
|---|---|
| SIMULSOL™ 165 : | 4% |
| MONTANOV™ 202 : | 1% |
| Caprylate-caprate triglyceride : | 15% |
| PECOSIL™ DCT : | 1% |
| Eau déminéralisée : | qsp. 100% |
| CAPIGEL™ 98 : | 0,5% |
| Latex inverse auto-inversible (1) : | 1% |
| PROTEOL™ OAT : | 2% |
| Hydroxyde de sodium : | qsp pH = 7 |

### Exemple 40 : Masque crème "rince off" restructurant pour cheveux stressés et fragilisés

**FORMULE**

| | |
|---|---|
| KETROL™T: | 0,5% |
| PECOSIL™ SPP50 : | 0,75% |
| N-cocoyl aminoacides : | 0,70% |
| Butylèneglycol : | 3,0% |
| Latex inverse auto-inversible (1) : | 3,0% |
| MONTANOV™ 82 : | 3,0% |
| Huile de jojoba : | 1,0% |
| LANOL™ P : | 6,0% |
| AMONYL™ DM : | 1,0% |
| LANOL™99 : | 5,0% |
| SEPICIDE™ HB | 0,3% |
| SEPICIDE™CI : | 0,2% |
| Parfum : | 0,2% |
| Eau: qsp | 100% |

### Exemple 36 : Crème solaire

| | |
|---|---|
| SIMULSOL™ 165 : | 3% |
| MONTANOV™ 202: | 2% |
| Benzoate C12-C15 : | 8% |
| PECOSIL™ PS 100 : | 2% |
| Diméthicone : | 2% |
| Cyclométhicone : | 5% |
| para-méthoxy cinnamate d'octyle : | 6% |
| Benzophénone-3 : | 4% |
| Oxyde de Titane : | 8% |
| Gomme xanthane : | 0,2% |
| Butylèneglycol : | 5% |
| Eau déminéralisée : | qsp.100% |
| Latex inverse auto-inversible (2) : | 1,5% |
| Conservateur, parfum : | qs |

### Exemple 37 : Gel de soin peaux mixtes

| | |
|---|---|
| Latex inverse auto-inversible (1) : | 4% |
| Squalane végétal : | 5% |
| Dimethicone : | 1,5% |
| SEPICONTROL™ A5 : | 4% |
| Gomme xanthane : | 0,3% |
| Eau : | qsp.100% |
| Conservateur, Parfum : | qs. |

### Exemple 38 : Lotion capillaire

**FORMULE**

| | |
|---|---|
| Butylène glycol : | 3,0% |
| Latex inverse auto-inversible (1) : | 3% |
| SIMULSOL™1293 : | 3,0% |
| Acide lactique : | qs. pH = 6 |
| SEPICIDE™ HB: | 0,2% |
| SEPICIDE ™C1 : | 0,3% |
| Parfum : | 0,3% |
| Eau : | qsP. 100% |

### Exemple 39 : Shampooing protecteur et relaxant

**FORMULE**

| | |
|---|---|
| Amonyl™ 675 SB : | 5,0% |
| Sodium lauryl éther sulfate à 28% : | 35,0% |
| Latex inverse auto-inversible (2) : | 3,0% |
| SEPICIDE™ HB : | 0,5% |
| SEPICIDE™CI : | 0,3% |
| Hydroxyde de sodium : | QS pH = 7,2 |
| Parfum : | 0,3% |
| Colorant (FDC bleu 1/jaune 5) : | QS |
| Eau : QSP 100% | |

### Exemple 40 : Protecteur "leave-on" ; Soin Formule anti-stress pour cheveux

**FORMULE**

| | |
|---|---|
| KETROL™T : | 0,5% |
| mélange de cocoyl aminoacides : | 3,0% |
| Butylèneglycol : | 5,0% |
| DC 1501 : | 5,0% |
| Latex inverse auto-inversible (2) : | 4,0% |
| SEPICIDE™ HB : | 0,5% |
| SEPIC1 DE™CI : | 0,3% |
| Parfum : | 0,3% |
| Eau : | QSP 100% |

### Exemple 41 : Emulsion parfumante

**FORMULE**

| | |
|---|---|
| Isononyl Isononanoate : | 6% |
| Ethylhexylglycérine : | 1% |
| Easynov™ : | 2,5 % |
| Latex inverse auto-inversible (2) : | 3% |
| Aquaxyl™ : | 3% |
| Eau : | qsp 100% |
| Ethanol : | 40% |
| Parfum : | 2,0% |

### Exemple 42 : Emulsion déodorante

**FORMULE**

| | |
|---|---|
| Isononyl Isononanoate : | 6% |
| Fragrance : | 0,1% |
| Phenoxyéthanol & Ethylhexylglycérine : | 1% |
| Easynov™ : | 1.5% |
| Latex inverse auto-inversible (2) : | 2,5% |
| Lipacide™UG : | 1% |
| Eau : | qsp 100% |

### Exemple 43 : Emulsion antiperspirante

**FORMULE**

| | |
|---|---|
| Isononyl Isononanoate : | 6% |
| Ethylhexylglycérine : | 1% |
| Easynov™ : | 2,5% |
| Latex inverse auto-inversible (2) : | 0,5% |
| Aquaxyl™: | 3% |
| Eau : | qsp 100% |
| Aluminium chlorhydrate (50%) : | 30% |

Les définitions des produits commerciaux utilisés dans les exemples sont les suivantes : SIMULSOL™ 1293 est de l'huile de castor hydrogénée et polyéthoxylée, avec un indice d'éthoxylation égal à 40, commercialisé par la société SEPPIC.

CAPIGEL™ 98 est un épaississant liquide à base de copolymère acrylate commercialisé par la société SEPPIC.

KETROL™T est de la gomme de xanthane commercialisée par la société KELCO. LANOL™ 99 est de l'isononyl isononanoate commercialisé par la société SEPPIC. DC1501 est un mélange de cyclopentasiloxane et de diméthiconol commercialisé par la société DOW CHEMICAL.

MONTANOV™ 82 est un agent émulsionnant à base d'alcool cétéarylique et de cocoylglucos ide.

Le MONTANOV™ 68 (cétéaryl glucoside), est une composition auto-émulsionnable telle que décrite dans WO 92/06778, commercialisée par la société SEPPIC.

Le MICROPEARL™ M 100 est une poudre ultra fine au toucher très doux et à action matifiante commercialisée par la société MATSUMO.

Le SEPICIDE™ Cl, imidazolidine urée, est un agent conservateur commercialisé par la société SEPPIC.

PEMULEN™ TR est un polymère acrylique commercialisé par GOODRICH.

Le SIMULSOL™165 est du stéarate de glycérol auto-émulsionnable commercialisée par la société SEPPIC.

Le LANOL™ 1688 est un ester émollient à effet non gras commercialisé par la société SEPPIC.

Le LANOL™ 14M et le LANOL S sont des facteurs de consistance commercialisés par la société SEPPIC.

Le SEPICIDE™ HB, qui est un mélange de phénix éthanol, de méthyl parabène, d'éthyle parabène, de propylée parabène et de butyle parabène, est un agent conservateur commercialisé par la société SEPPIC.

Le MONTEINE™ CA est un agent hydratant commercialisé par la société SEPPIC.

Le SCHERCEMOL™ OP est un ester émollient à effet non gras.

Le LANOL™ P est un additif à effet stabilisant commercialisé par la société SEPPIC.

Le PARSOL™ MCX est du (para-méthoxy) cinnamate d'octyle; commercialisé par la société GIVAUDAN.

Le SEPIPERL™N est un agent nacrant, commercialisé par la société SEPPIC, à base d'un mélange d'alkyl poly glucosides tels que ceux décrits dans WO 95/13863.

Le MICROPEARL™ SQL est un mélange de micro particules renfermant du Scalante qui se libère sous l'action du massage; il est commercialisé par la société MATSUMO.

Le LANOL™ 99 est de l'isononanoate d'isononyle commercialisé par la société SEPPIC.

Le LANOL™ 37T est du Triheptonate de glycérol, commercialisé par la société SEPPIC.

Le SOLAGUM™ L est un Carraghénane commercialisé par la société SEPPIC.

Le MARCOL™ 82 est une huile de paraffine commercialisée par la société EXXON. Le LANOL™ 84D est du malte de dactyle commercialisé par la société SEPPIC. Le PARSOL NOX™ est un filtre solaire commercialisé par la société GIVAUDAN.

EUSOLEX™ 4360 est un filtre solaire commercialisé par la société MERCK.

Le DOW CORNING™ 245 FLuid est de la Cyclométhicone, commercialisée par la société DOW CORNING.

Le LIPACIDE™ PVB, est un hydrolysat de protéines de blé acyle commercialisé par la société SEPPIC.

Le MICROPEARL™ LM est un mélange de Squalane, de polyméthylméthacrylate et de menthol, commercialisé par la société SEPPIC.

Le SEPICONTROL™ A5 est un mélange capryloyl glycine, sarcosine, extrait de cinnamon zylanicum, commercialisé par la société SEPPIC, tel que ceux décrits dans la demande internationale de brevet PCT/FR98/01313 déposée le 23 juin 1998.

Le LANOL™ 2681 est un mélange caprylate, caprate de coprah, commercialisé par la société SEPPIC.

Le MONTANOV™ 202, est une composition APG/alcools gras telle que décrite dans WO9 98/47610, commercialisée par la société SEPPIC.

L'EASYNOV™ (nom INCI : Octyldodecanol, Octyldodecyl Xyloside and PEG-30 Dipolyhydroxystearate) est une composition émulsionnante commercialisée par la société SEPPIC.

L'AQUAXYL™ (INCI name : Xylitylglucoside & Anhydroxylitol & Xylitol) est un agent hydratant commercialisé par la société SEPPIC.

Le LIPACIDE™UG (INCI name : undecylenoyl glycine) est un agent déodorant et dermo-purifiant commercialisé par la société SEPPIC.

## Revendications

1. Composition sous forme d'un latex inverse auto-inversible comprenant pour 100% de sa masse :
**a) - De 25% massique à 80% massique** d'un polyélectrolyte anionique (P) réticulé, issu de la polymérisation pour 100% molaire :
**i) -** d'une proportion molaire supérieure ou égale à 30% et inférieure ou égale à 95% d'unités monomériques issues d'au moins un monomère comportant une fonction acide fort, libre partiellement ou totalement salifiée ;
**ii) - optionnellement** d'une proportion molaire supérieure ou égale à 10% et inférieure ou égale à 65% d'unités monomériques issues d'au moins un monomère neutre
**iii) - optionnellement** d'une proportion molaire supérieure ou égale à 5% et inférieure ou égale à 65% d'unités monomériques issues d'au moins un monomère comportant une fonction acide faible, libre partiellement ou totalement salifiée ;
et
**iv) -** d'une proportion molaire supérieure à 0% et inférieure ou égale à 5% d'au moins un monomère de réticulation diéthylénique ou polyéthylénique;
**b) - De 0,5% à 10% massique** d'un terpolymère de méthacrylate de stéaryle, de méthacrylate de béhènyle pentacosaéthoxylé et de N,N-diméthyl acrylamide, issu de la polymérisation pour 100% molaire :
de 80% molaire à 95% molaire de méthacrylate de stéaryle,
de 2,5% molaire à 10% molaire de N,N-diméthyl acrylamide, et
de 2,5% molaire à 10% molaire de méthacrylate de béhènyle pentacosaéthoxylé,
**c) - De 5% massique à 40% massique** d'au moins une huile, et
**d) - De 0,1% massique à 40% massique** d'eau ;
ladite composition ne comprenant en son sein, ni d'agents tensioactifs de type huile-dans-eau, ni d'agents tensioactifs de type eau-dans-huile.

2. Composition telle que définie à la revendication 1, dans laquelle le terpolymère de méthacrylate de stéaryle, de méthacrylate de béhènyle pentacosaéthoxylé et de N,N-diméthyl acrylamide, est choisi parmi les terpolymères suivants :
- Terpolymère de méthacrylate de stéaryle, de méthacrylate de béhènyle pentacosaéthoxylé et de N,N-diméthyl acrylamide [ratio molaire (A)/(B) /(C) = 87/5/8];
- Terpolymère de méthacrylate de stéaryle, de méthacrylate de béhènyle pentacosaéthoxylé et de N,N-diméthyl acrylamide [ratio molaire (A)/(B)/(C) = 80/5/15] ;
- Terpolymère de méthacrylate de stéaryle, de méthacrylate de béhènyle pentacosaéthoxylé et de N,N-diméthyl acrylamide [ratio molaire (A)/(B) /(C) = 85/10/5] ;
- Terpolymère de méthacrylate de stéaryle, de méthacrylate de béhènyle pentacosaéthoxylé et de N,N-diméthyl acrylamide [ratio molaire (A)/(B)/(C) = 80/10/10] ;
- Terpolymère de méthacrylate de stéaryle, de méthacrylate de béhènyle pentacosaéthoxylé et de N,N-diméthyl acrylamide [ratio molaire (A)/(B)/(C) = 90/5/5] ; ou du,
- Terpolymère de méthacrylate de stéaryle, de méthacrylate de béhènyle pentacosaéthoxylé et de N,N-diméthyl acrylamide [ratio molaire (A)/(B)/(C) = 95/2,5/2,5)].

3. Composition telle que définie à l'une des revendications 1 ou 2, pour laquelle dans ledit polyélectrolyte anionique P, les unités monomériques comportant une fonction acide fort sont issues de l'acide 2-méthyl 2-[(1-oxo 2-propènyl) amino] 1-propanesulfonique partiellement ou totalement salifié en sel de sodium, en sel de potassium, ou en sel d'ammonium.

4. Composition telle que définie à l'une quelconque des revendications 1 à 3, dans laquelle, le ratio massique entre ledit terpolymère de méthacrylate de stéaryle, de méthacrylate de béhènyle pentacosaéthoxylé et de N,N-diméthyl acrylamide et ledit polyélectrolyte anionique (P) est supérieur ou égal à 1/100 et inférieur ou égal à 1/5.

5. Composition telle que définie à l'une quelconque des revendications 1 à 4, sous forme d'un latex inverse auto-inversible comprenant pour 100% de sa masse :
**a) - De 25% massique à 50% massique** d'un polyélectrolyte anionique (P) branché ou réticulé, issu de la polymérisation pour 100% molaire :
**i) -** d'une proportion molaire supérieure ou égale à 30% et inférieure ou égale à 95% d'unités monomériques issues d'au moins un monomère comportant une fonction acide fort, libre partiellement ou totalement salifiée ;
**ii) -** d'une proportion molaire supérieure ou égale à 10% et inférieure ou égale à 65% d'unités monomériques issues d'au moins un monomère neutre
et
**iv) -** d'une proportion molaire supérieure à 0% et inférieure ou égale à 5% d'au moins un monomère de réticulation diéthylénique ou polyéthylénique;
**b) - De 1% à 5% massique** d'un terpolymère de méthacrylate de stéaryle, de méthacrylate de béhènyle pentacosaéthoxylé et de N,N-diméthyl acrylamide, issu de la polymérisation pour 100% molaire :
de 80% molaire à 95% molaire de méthacrylate de stéaryle,
de 2,5% molaire à 10% molaire de N,N-diméthyl acrylamide et
de 2,5% molaire à 10% molaire de méthacrylate de béhènyle pentacosaéthoxylé,
**c) - De 10% massique à 30% massique** d'au moins une huile, et
**d) - De 20% massique à 40% massique** d'eau.

6. Composition telle que définie à l'une quelconque des revendications 1 à 4, sous forme d'un latex inverse auto-inversible comprenant pour 100% de sa masse :
**a) - De 50% massique à 80% massique** d'un polyélectrolyte anionique (P) branché ou réticulé, issu de la polymérisation pour 100% molaire :
**i) -** d'une proportion molaire supérieure ou égale à 30% et inférieure ou égale à 95% d'unités monomériques issues d'au moins un monomère comportant une fonction acide fort, libre partiellement ou totalement salifiée ;
**ii) -** d'une proportion molaire supérieure ou égale à 10% et inférieure ou égale à 65% d'unités monomériques issues d'au moins un monomère neutre
et
**iv) -** d'une proportion molaire supérieure à 0% et inférieure ou égale à 5% d'au moins un monomère de réticulation diéthylénique ou polyéthylénique;
**b) - De 1% à 10% massique** d'un terpolymère de méthacrylate de stéaryle, de méthacrylate de béhènyle pentacosaéthoxylé et de N,N-diméthyl acrylamide, issu de la polymérisation pour 100% molaire :
de 80% molaire à 95% molaire de méthacrylate de stéaryle,
de 2,5% molaire à 10% molaire de N,N-diméthyl acrylamide et
de 2,5% molaire à 10% molaire de méthacrylate de béhènyle pentacosaéthoxylé,
**c) - De 10% massique à 40% massique** d'au moins une huile, et
**d) - Au plus 5% massique** d'eau.

7. Procédé de préparation de la composition telle que définie précédemment, **caractérisé en ce que** :
a) l'on émulsionne une phase aqueuse (A) contenant les monomères et les éventuels additifs hydrophiles, dans une phase organique (O) contenant ledit terpolymère de méthacrylate de stéaryle, de méthacrylate de béhènyle pentacosaéthoxylé et de N,N-diméthyl acrylamide, l'huile ou le mélange d'huiles un mélange constitué des huiles destinées à être présente dans la composition finale et si nécessaire une ou plusieurs huiles volatiles ainsi que les éventuels additifs hydrophobes,
b) l'on amorce la réaction de polymérisation par introduction dans l'émulsion formée en a), d'un initiateur de radicaux libres puis on la laisse se dérouler, et si désiré,
c) l'on concentre par distillation le milieu réactionnel issu de l'étape b), jusqu'à élimination complète de ladite huile volatile.

8. Utilisation du terpolymère de méthacrylate de stéaryle, de méthacrylate de béhènyle pentacosaéthoxylé et de N,N-diméthyl acrylamide, issu de la polymérisation pour 100% molaire :
de 80% molaire à 95% molaire de méthacrylate de stéaryle,
de 2,5% molaire à 10% molaire de N,N-diméthyl acrylamide et
de 2,5% molaire à 10% molaire de méthacrylate de béhènyle pentacosaéthoxylé, comme agent stabilisant de l'émulsion eau dans huile comprenant l'ensemble des constituants nécessaires à la préparation de la composition sous forme d'un latex inverse auto-inversible telle que définie à l'une quelconque des revendications 1 à 6, émulsion eau dans huile au sein de laquelle se déroule la réaction de polymérisation en émulsion inverse.

9. Composition topique cosmétique, dermopharmaceutique ou pharmaceutique, **caractérisée en ce qu'**elle comprend, comme agent épaississant et/ou émulsionnant, une quantité efficace de la composition telle que définie à l'une quelconque des revendications 1 à 6.

10. Utilisation de la composition sous forme de latex inverse auto-inversible, telle que définie à l'une quelconque des revendications 1 à 6, pour épaissir une composition topique cosmétique, dermopharmaceutique ou pharmaceutique comprenant au moins une phase aqueuse.

## Patentansprüche

1. Zusammensetzung in Form eines inversen autoinversiblen Latex, umfassend, für 100 % ihrer Masse:
a) - von 25 Massenprozent bis 80 Massenprozent eines vernetzten anionischen Polyelektrolyts (P), aus der Polymerisierung für 100 Molprozent:
i) eines Molverhältnisses größer oder gleich 30 % und kleiner oder gleich 95 % von Monomereinheiten, die von mindestens einem Monomer stammen, das eine starke, freie, teilweise oder vollständig salzhaltige Säurefunktion umfasst;
ii) optional eines Molverhältnisses größer oder gleich 10 % und kleiner oder gleich 65 % von Monomereinheiten, die aus mindestens einem neutralen Monomer stammen
iii) optional eines Molverhältnisses größer oder gleich 5 % und kleiner oder gleich 65 % von Monomereinheiten, die von mindestens einem Monomer stammen, das eine schwache, freie, teilweise oder vollständig salzhaltige Säurefunktion umfasst; und
iv) eines Molverhältnisses größer 0 % und kleiner oder gleich 5 % mindestens eines Diethylen- oder Polyethylen-Vernetzungsmonomers;
b) - von 0,5 bis 10 Massenprozent eines Stearylmethacrylat-, pentacosaethoxylierten Behenylmethacrylat- und N,N-dimethylacrylamid-Terpolymers aus der Polymerisierung für 100 Molprozent:
von 80 bis 95 Molprozent Stearylmethacrylat,
von 2,5 bis 10 Molprozent N,N-dimethylacrylamid, und
von 2,5 bis 10 Molprozent pentacosaethoxyliertem Behenylmenthacrylat,
c) - von 5 Massenprozent bis 40 Massenprozent mindestens eines Öls, und
d) - von 0,1 Massenprozent bis 40 Massenprozent Wasser; wobei die Zusammensetzung weder oberflächenaktive Mittel vom Typ ÖL-in-Wasser noch oberflächenaktive Mittel vom Typ Wasser-in-Öl umfasst.

2. Zusammensetzung wie in Anspruch 1 definiert, wobei das Stearylmethacrylat-, das pentacosaethoxylierte Behenylmethacrylat- und das N,N-dimethylacrylamid-Terpolymer ausgewählt ist aus den folgenden Terpolymeren:
- Stearylmethacrylat-, pentacosaethoxyliertes Behenylmethacrylat- und N,N-dimethylacrylamid-Terpolymer [molares Verhältnis (A)/(B)/(C) = 87/5/8];
- Stearylmethacrylat-, pentacosaethoxyliertes Behenylmethacrylat- und N,N-dimethylacrylamid-Terpolymer [molares Verhältnis (A)/(B)/(C) = 80/5/15];
- Stearylmethacrylat-, pentacosaethoxyliertes Behenylmethacrylat- und N,N-dimethylacrylamid-Terpolymer [molares Verhältnis (A)/(B)/(C) = 85/10/5];
- Stearylmethacrylat-, pentacosaethoxyliertes Behenylmethacrylat- und N,N-dimethylacrylamid-Terpolymer [molares Verhältnis (A)/(B)/(C) = 80/10/10];
- Stearylmethacrylat-, pentacosaethoxyliertes Behenylmethacrylat- und N,N-dimethylacrylamid-Terpolymer [molares Verhältnis (A)/(B)/(C) = 90/5/5];oder
- Stearylmethacrylat-, pentacosaethoxyliertes Behenylmethacrylat- und N,N-dimethylacrylamid-Terpolymer [molares Verhältnis (A)/(B)/(C) = 95/2,5/2,5].

3. Zusammensetzung wie in Anspruch 1 oder 2 definiert, wobei in dem anionischen Polyelektrolyt P die Monomereinheiten, die eine starke Säurefunktion umfassen, aus der 2-Methyl 2-[(1-oxo 2-propenyl) amino] 1-propansulfonsäure stammen, die teilweise oder vollständig salzhaltig ist mit Natriumsalz, Kaliumsalz oder Ammoniumsalz.

4. Zusammensetzung wie in einem der Ansprüche 1 bis 3 definiert, wobei das Massenverhältnis zwischen dem Stearylmethacrylat-, pentacosaethoxylierten Behenylmethacrylat- und dem N,N-dimethylacrylamid-Terpolymer und dem anionischen Polyelektrolyt (P) größer oder gleich 1/100 und kleiner oder gleich 1/5 ist.

5. Zusammensetzung wie in einem der Ansprüche 1 bis 4 definiert, in Form eines inversen autoinversiblen Latex, umfassend, für 100 % ihrer Masse:
a) - von 25 Massenprozent bis 50 Massenprozent eines verzweigten oder vernetzten anionischen Polyelektrolyts (P) aus der Polymerisierung für 100 Molprozent:
i) eines Molverhältnisses größer oder gleich 30 % und kleiner oder gleich 95 % von Monomereinheiten die von mindestens einem Monomer stammen, das eine starke, freie, teilweise oder vollständig salzhaltige Säurefunktion umfasst;
ii) eines Molverhältnisses größer oder gleich 10 % und kleiner oder gleich 65 % von Monomereinheiten aus mindestens einem neutralen Monomer und
iv) eines Molverhältnisses größer 0 % und kleiner oder gleich 5 % mindestens eines Diethylen- oder Polyethylen-Vernetzungsmonomers;
b) - von 1 bis 5 Massenprozent eines Stearylmethacrylat-, pentacosaethoxylierten Behenylmethacrylat- und N,N-dimethylacrylamid-Terpolymers aus der Polymerisierung für 100 Molprozent:
von 80 bis 95 Molprozent Stearylmethacrylat,
von 2,5 bis 10 Molprozent N,N-dimethylacrylamid, und
von 2,5 bis 10 Molprozent pentacosaethoxyliertem Behenylmenthacrylat,
c) - von 10 Massenprozent bis 30 Massenprozent mindestens eines Öls, und
d) - von 20 Massenprozent bis 40 Massenprozent Wasser;

6. Zusammensetzung wie in einem der Ansprüche 1 bis 4 definiert, in Form eines inversen autoinversiblen Latex, umfassend, für 100 % ihrer Masse:
a) - von 50 Massenprozent bis 80 Massenprozent eines verzweigten oder vernetzten anionischen Polyelektrolyts (P), aus der Polymerisierung für 100 Molprozent:
i) eines Molverhältnisses größer oder gleich 30 % und kleiner oder gleich 95 % von Monomereinheiten die von mindestens einem Monomer stammen, das eine starke, freie, teilweise oder vollständig salzhaltige Säurefunktion umfasst;
ii) eines Molverhältnisses größer oder gleich 10 % und kleiner oder gleich 65 % von Monomereinheiten aus mindestens einem neutralen Monomer und
iv) eines Molverhältnisses größer 0 % und kleiner oder gleich 5 % mindestens eines Diethylen- oder Polyethylen-Vernetzungsmonomers;
b) - von 1 bis 10 Massenprozent eines Stearylmethacrylat-, pentacosaethoxylierten Behenylmethacrylat- und N,N-dimethylacrylamid-Terpolymers aus der Polymerisierung für 100 Molprozent:
von 80 bis 95 Molprozent Stearylmethacrylat,
von 2,5 bis 10 Molprozent N,N-dimethylacrylamid, und
von 2,5 bis 10 Molprozent pentacosaethoxyliertem Behenylmenthacrylat,
c) - von 10 Massenprozent bis 40 Massenprozent mindestens eines Öls, und
d) - höchstens 5 Massenprozent Wasser.

7. Verfahren zur Herstellung der Zusammensetzung, wie oben definiert, **dadurch gekennzeichnet, dass**
a) eine wässrige Phase (A), die die Monomere und die eventuellen hydrophilen Zusatzstoffe enthält, in einer organischen Phase (O) emulgiert wird, enthaltend das Stearylmethacrylat-, das pentacosaethoxylierte Behenylmethacrylat- und das N,N-dimethylacrylamid-Terpolymer, das Öl und die Mischung von Ölen, eine Mischung, die aus Ölen besteht, die ausgelegt sind, um in der endgültigen Zusammensetzung vorhanden zu sein, und, falls erforderlich, ein oder mehrere flüchtige Öle, ebenso wie die eventuellen hydrophoben Zusatzstoffe,
b) die Polymerisierungsreaktion durch Einführung in die Emulsion gestartet wird, gebildet aus s) einem Initiator mit freien Radikalen, sie dann ablaufen gelassen wird, und, falls gewünscht,
c) das Reaktionsmedium aus Schritt b) durch Destillierung bis zur vollständigen Beseitigung des flüchtigen Öls konzentriert wird.

8. Verwendung des Stearylmethacrylat-, pentacosaethoxylierten Behenylmethacrylat- und N,N-dimethylacrylamid-Terpolymers aus der Polymerisierung für 100 Molprozent:
von 80 bis 95 Molprozent Stearylmethacrylat,
von 2,5 bis 10 Molprozent N,N-dimethylacrylamid, und
von 2,5 bis 10 Molprozent pentacosaethoxyliertem Behenylmenthacrylat,
als Stabilisierungsmittel der Emulsion Wasser-in-Öl, umfassend die Gesamtheit der Bestandteile, die zur Herstellung der Zusammensetzung in Form eines inversen autoinversiblen Latex erforderlich sind, wie in einem der Ansprüche 1 bis 6 definiert, einer Emulsion Wasser-in-Öl, in der die Polymerisierungsreaktion in inverser Emulsion abläuft.

9. Kosmetische, dermopharmazeutische oder pharmazeutische topische Zusammensetzung, **dadurch gekennzeichnet, dass** sie als Verdickungsmittel und/oder Emulsionsmittel eine wirksame Menge der Zusammensetzung umfasst, wie in einem der Ansprüche 1 bis 6 definiert.

10. Verwendung der Zusammensetzung in Form eines inversen autoinversiblen Latex, wie in einem der Ansprüche 1 bis 6 definiert, um eine kosmetische, dermopharmazeutische oder pharmazeutische topische Zusammensetzung zu verdicken, umfassend mindestens eine wässrige Phase.

## Claims

1. Composition in the form of a self-reversible reverse latex comprising, for 100% of its mass:
a) - from 25% by mass to 80% by mass of a crosslinked anionic polyelectrolyte (P), issuing from the polymerisation, for 100% molar, of:
i) - a molar proportion greater than or equal to 30% and less than or equal to 95% of monomeric units issuing from at least one monomer comprising a partially or totally salified free strong acid function;
ii) - optionally a molar proportion greater than or equal to 10% and less than or equal to 65% of monomeric units issuing from at least one neutral monomer;
iii) - optionally a molar proportion greater than or equal to 5% and less than or equal to 65% of monomeric units issuing from at least one monomer comprising a partially or totally salified free weak acid function; and
iv) - a molar proportion greater than 0% and less than or equal to 5% of at least one diethylene or polyethylene crosslinking monomer;
b) - from 0.5% to 10% by mass of a terpolymer of stearyl methacrylate, pentacosaethoxylated behenyl methacrylate and N,N-dimethyl acrylamide, issuing from the polymerisation, for 100% molar, of:
from 80% molar to 95% molar of stearyl methacrylate,
from 2.5% molar to 10% molar of N,N-dimethyl acrylamide, and
from 2.5% molar to 10% molar of pentacosaethoxylated behenyl methacrylate,
c) - from 5% by mass to 40% by mass of at least one oil, and
d) - from 0.1% to 40% by mass of water;
said composition comprising in it neither surfactants of the oil-in-water type nor surfactants of the water-in-oil type.

2. Composition as defined in claim 1, in which the terpolymer of stearyl methacrylate, pentacosaethoxylated behenyl methacrylate and N,N-dimethyl acrylamide is chosen from the following terpolymers:
- Terpolymer of stearyl methacrylate, pentacosaethoxylated behenyl methacrylate and N,N-dimethyl acrylamide [molar ratio (A)/(B)/(C) = 87/5/8];
- Terpolymer of stearyl methacrylate, pentacosaethoxylated behenyl methacrylate and N,N-dimethyl acrylamide [molar ratio (A)/(B)/(C) = 80/5/15];
- Terpolymer of stearyl methacrylate, pentacosaethoxylated behenyl methacrylate and N,N-dimethyl acrylamide [molar ratio (A)/(B)/(C) = 85/10/5];
- Terpolymer of stearyl methacrylate, pentacosaethoxylated behenyl methacrylate and N,N-dimethyl acrylamide [molar ratio (A)/(B)/(C) = 80/10/10];
- Terpolymer of stearyl methacrylate, pentacosaethoxylated behenyl methacrylate and N,N-dimethyl acrylamide [molar ratio (A)/(B)/(C) = 90/5/5];
- Terpolymer of stearyl methacrylate, pentacosaethoxylated behenyl methacrylate and N,N-dimethyl acrylamide [molar ratio (A)/(B)/(C) = 95/2.5/2.5].

3. Composition as defined in one of claims 1 or 2, for which, in said anionic polyelectrolyte P, the monomeric units comprising a strong acid function issue from 2-methyl 2-[(1-oxo 2-propenyl) amino] 1-propanesulfonic acid, partially or totally salified in sodium salt, potassium salt or ammonium salt.

4. Composition as defined in any one of claims 1 to 3, in which the ratio by mass between said terpolymer of stearyl methacrylate, pentacosaethoxylated behenyl methacrylate and N,N-dimethyl acrylamide and said anionic polyelectrolyte (P) is greater than or equal to 1/100 and less than or equal to 1/5.

5. Composition as defined in any one of claims 1 to 4, in the form of a self-reversible reverse latex comprising, for 100% of its mass:
a) - from 25% by mass to 50% by mass of a branched or crosslinked anionic polyelectrolyte (P), issuing from the polymerisation, for 100% molar, of:
i) - a molar proportion greater than or equal to 30% and less than or equal to 95% of monomeric units issuing from at least one monomer comprising a partially or totally salified free strong acid function;
ii) - a molar proportion greater than or equal to 10% and less than or equal to 65% of monomeric units issuing from at least one neutral monomer; and
iv) - a molar proportion greater than 0% and less than or equal to 5% of at least one diethylene or polyethylene crosslinking monomer;
b) - from 1% to 5% by mass of a terpolymer of stearyl methacrylate, pentacosaethoxylated behenyl methacrylate and N,N-dimethyl acrylamide, issuing from the polymerisation, for 100% molar, of:
from 80% molar to 95% molar of stearyl methacrylate,
from 2.5% molar to 10% molar of N,N-dimethyl acrylamide, and
from 2.5% molar to 10% molar of pentacosaethoxylated behenyl methacrylate,
c) - from 10% by mass to 30% by mass of at least one oil, and
d) - from 20% to 40% by mass of water.

6. Composition as defined in any one of claims 1 to 4, in the form of a self-reversible reverse latex comprising, for 100% of its mass:
a) - from 50% by mass to 80% by mass of a branched or crosslinked anionic polyelectrolyte (P), issuing from the polymerisation, for 100% molar, of:
i) - a molar proportion greater than or equal to 30% and less than or equal to 95% of monomeric units issuing from at least one monomer comprising a partially or totally salified free strong acid function;
ii) - a molar proportion greater than or equal to 10% and less than or equal to 65% of monomeric units issuing from at least one neutral monomer; and
iv) - a molar proportion greater than 0% and less than or equal to 5% of at least one diethylene or polyethylene crosslinking monomer;
b) - from 1% to 10% by mass of a terpolymer of stearyl methacrylate, pentacosaethoxylated behenyl methacrylate and N,N-dimethyl acrylamide, issuing from the polymerisation, for 100% molar, of:
from 80% molar to 95% molar of stearyl methacrylate,
from 2.5% molar to 10% molar of N,N-dimethyl acrylamide, and
from 2.5% molar to 10% molar of pentacosaethoxylated behenyl methacrylate,
c) - from 10% by mass to 40% by mass of at least one oil, and
d) - no more than 5% by mass of water.

7. Method for preparing a composition as previously defined, **characterised in that**:
a) an aqueous phase (A) containing the monomers and optional hydrophilic additives is emulsified, in an organic phase (O) containing said terpolymer of stearyl methacrylate, pentacosaethoxylated behenyl methacrylate and N,N-dimethyl acrylamide, the oil or the mixture of oils, a mixture consisting of oils intended to be present in the final composition, and if necessary one or more volatile oils, as well as the optional hydrophobic additives,
b) the polymerisation reaction is initiated by introducing a free-radical initiator into the emulsion formed at a), and it is then left to take place, and, if desired,
c) it is concentrated by distilling the reaction medium issuing from step b), to complete elimination of said volatile oil.

8. Use of the terpolymer of stearyl methacrylate, pentacosaethoxylated behenyl methacrylate and N,N-dimethyl acrylamide, issuing from the polymerisation, for 100% molar, of:
from 80% molar to 95% molar of stearyl methacrylate,
from 2.5% molar to 10% molar of N,N-dimethyl acrylamide, and
from 2.5% molar to 10% molar of pentacosaethoxylated behenyl methacrylate, as a stabilising agent for the water-in-oil emulsion, comprising all the constituents necessary for preparing the composition in the form of a self-reversible reverse latex as defined in any one of claims 1 to 6, a water-in-oil emulsion in which the reverse-emulsion polymerisation reaction takes place.

9. Cosmetic, dermopharmaceutical or pharmaceutical topical composition, **characterised in that** it comprises, as a thickening agent and/or emulsifier, an effective quantity of the composition as defined in any one of claims 1 to 6.

10. Use of the composition in the form of self-reversible reverse latex, as defined in any one of claims 1 to 6, for thickening a cosmetic, dermopharmaceutical or pharmaceutical topical composition, comprising at least one aqueous phase.
